# EUROPEAN PATENT APPLICATION

(11) **EP 1 541 117 A1**
(43) Date of publication of application: **15.06.2005**
(21) Application number: 03028495.4
(22) Date of filing: 12.12.2003
(51) Int. Cl.: A61K 7/06

(54) **Cosmetic rinse-off compositions comprising inulin-type fructan**

(71) Applicant: Tiense Suikerraffinaderij N.V., 1150 Brussel (BE)
(72) Inventor: Levecke, Bart, 1981 Hofstade (BE); Booten, Karl, 3450 Geebets (BE)
(74) Representative: Hermans, Johny

(57) **Abstract**

The use is disclosed of an inulin-type fructan for the manufacture of cosmetic rinse-off compositions for the treatment of the hair and/or the skin. The compositions comprise, apart from conventional ingredients in conventional amounts, from 0.10 to 10% of the inulin-type fructan, and are free from (i) cationic polymers, (ii) terpolymers comprising a non-ionic monomer bearing a urethane group, and (iii) polysaccharides that are different from inulin-type fructan. Typical rinse-off compositions in accordance with the invention are also free from monosaccharides and from di- and oligosaccharides that are different from inulin-type fructan, apart from the mono- and disaccharides that naturally occur in inulin-type fructan.

The inulin-type fructan, which is biodegradable and has no eutrophic effects on surface waters, can be used to completely substitute cationic polymeric conditioning agents in conventional cosmetic rinse-off compositions.

Novel rinse-off compositions are also disclosed.

## Description

### Field of the invention

The present invention relates to cosmetic rinse-off compositions comprising an inulin-type fructan, to a process for the manufacture of same, as well as to the use of an inulin-type fructan as a total substitute for cationic polymeric conditioning agents in cosmetic rinse-off compositions.

### Background and prior art

So-called rinse-off compositions constitute an economically important class of cosmetics. Rinse-off compositions are aqueous compositions that are intended for the treatment of the hair and/or the skin, that subsequent to their application are rinsed-off with water while leaving a deposit of one or more ingredients on the hair and/or the skin in an amount that provides desirable cosmetic effects. Typical rinse-off compositions include hair-shampoos, hair-conditioners, shower-shampoos, shower-gels and bath-foams. Key features of rinse-off compositions include cleaning effects and lathering effects, and, when intended for the treatment of hair, the provision of conditioning effects, and when intended for the treatment of the skin, the provision of a soft and smooth feeling. Further features of importance include viscosity, homogeneity and stability of the rinse-off composition.

So, the compositions of rinse-off cosmetics are largely determined by the intended application. Accordingly, a rinse-off composition contains various ingredients in order to provide desired physico-chemical properties to the rinse-off composition and to provide desired cosmetic properties and effects for the intended application.

Herein components of conventional cosmetic rinse-off compositions are named ingredients.

A first class of ingredients is constituted by cleaning (cleansing) agents, including soaps and detergents. The latter include anionic, non-ionic and amphoteric surfactants and mixtures thereof. The cleaning agents having pronounced tensio-active and lather-forming properties, provide a rich foam and promote efficient removal from the hair and skin of contaminants, such as dust, natural fats and residues of transpiration.

A second class of ingredients is constituted by conditioning agents, which are essential ingredients of rinse-off compositions for the treatment of the hair. Conditioning agents minimise static electricity in the hair and make that the hair is easily manageable, easily disentangling, and that the combability of the wet and the dry hair is easy and smooth. They furthermore, provide body, lustre and softness to the hair. These and other common cosmetic effects are conventionally referred to, also herein, as conditioning properties or conditioning effects.

Conditioning agents are cationic-type, anionic-type or non-ionic-type compounds, referred to herein as cationic-, anionic-, and non-ionic conditioning agents, that leave, when the cosmetic composition is rinsed off, a deposit on the hair that provides to the hair desired conditioning properties.

Cationic conditioning agents are typically cationic polymers, namely polymeric compounds that contain cationic groups and/or groups that are ionisable in aqueous medium to form cationic groups. Cationic groups are, for example, groups that bear a quaternary nitrogen atom. Accordingly, polymers bearing the latter cationic groups are often named polyquaternium compounds.

By the term cationic polymers is meant herein cationic polymeric conditioning agents.

Hair is negatively charged and due to their opposite electrical charge vis-à-vis the hair, cationic polymers easily interact with the hair and leave a deposit on it when cosmetic compositions containing said cationic polymers are rinsed off. The deposit of the cationic polymer on the hair provides the conditioning properties. Besides, cationic polymers are readily soluble in water-based shampoo compositions, which facilitates the preparation of the rinse-off compositions.

Anionic conditioning agents typically consist of so-called anionic polymers, namely polymeric compounds that contain units bearing one or more carboxylate groups. Typical anionic conditioning agents are for example alginate salts.

Non-ionic conditioning agents consist of non-ionic-type compounds, including high molecular weight compounds and certain polymeric compounds. Typical non-ionic-type conditioning agents comprise various waxy and oily materials, silicones and silicone-containing copolymers.

Due to their negative electrical charge and absence of electrical charge, respectively, anionic-type compounds and non-ionic-type compounds are commonly less effective conditioning agents compared to cationic-type compounds.

In view of their performance and solubility in water, cationic polymers are preferably used as conditioning agent and, at present, about 75 per cent of the conventional cosmetic rinse-off compositions contain cationic polymers.

Typical cationic polymers used as conditioning agent in rinse-off compositions are disclosed in WO 02/055036.

Commercially available cationic polymers include copolymers of vinyl-pyrrolidone and dimethylaminoethyl methacrylate quaternised with dimethyl sulfate (available as Gafquat®, trade name of International Specialty Products), homopolymers of dimethyldiallylammonium chloride and copolymers of dimethyldiallylammonium chloride and acrylamide (available as Merquat®, trade name of ONDEO Nalco), and (trimethyl-ammonium propyl ether) chloride derivatives of cellulose (available as UCAREF™ Polymers, trade name of Amerchol).

A further class of ingredients, is constituted by so-called thickeners or viscosity improvers, namely ingredients that provide to the cosmetic rinse-off composition a desired viscosity, so that the composition appears, for example, in the form of a gel or cream, a viscous liquid, a pourable or a free flowing liquid.

Furthermore, thickeners are also used to give physical stability to the rinse-off composition. In this respect, stability refers to the maintenance of the homogeneity of the composition. The thickener largely prevents the composition of separating into different liquid layers in case the composition is present in the form of an emulsion, prevents settling of a solid phase in case the composition is present in the form of a suspension, or prevents crystallisation of some of the ingredients. Typically used thickeners include xanthan gums, alginate salts, and cross-linked acrylic (co)-polymers, for example Tego Carbomer® (trade name of Degussa Care Specialties), and Carbopol® (trade name of Noveon).

Cosmetic rinse-off compositions furthermore commonly comprise various additional ingredients, for example colouring agents, perfuming agents, anti-bacterial agents, pH-stabilising agents, anti-oxidants, and stabilisers to inhibit darkening resulting from chemical reactions of certain ingredients.

So, desirable properties of a cosmetic rinse-off composition include on the one hand physico-chemical features, particularly an appropriate viscosity, physical stability, and good rinse-off properties, and on the other hand cosmetic effects on the skin and/or the hair, such as smooth feeling of the skin, and easy disentangling, soft feeling and good conditioning of the hair.

In the search for ingredients that provide said and other desirable properties to rinse-off compositions, various ingredients and combinations of ingredients have been evaluated, and miscellaneous rinse off compositions have already been disclosed.

For example, certain saccharides and polysaccharides have been disclosed for providing desirable cosmetic effects on the hair or the skin, particularly softness and smooth feel, whereas certain polysaccharides, particularly starches and modified starches, pectins, gums, and other polymeric compounds, particularly acrylic polymers, have been disclosed as thickeners.

However, the use of most of these ingredients and combinations thereof, often resulted in compositions that present on the one hand desirable cosmetic properties but on the other hand insufficient physico-chemical properties and/or other disadvantages, or *vise versa.*

Representative prior art compositions are given below. All percentages(%)indicated are in per cent by weight, unless otherwise stipulated.

US 2, 237,629, discloses a shampoo composed of 25% ammonium salt of the sulphonic ester of monoethanolamide of palmitic acid, 10% saccharide (cane sugar), 65% water, and suitable amounts of perfume and/or colouring matter.

US 3,998,761 discloses shampoo compositions comprising, a conventional detergent system, typically a synthetic sulfonate or a synthetic non-ionic detergent, and 4 to 20% of beer solids as conditioning agents. The beer solids are mainly composed of a mixture of proteins and polydisperse polysaccharides containing upward of one glucose unit per molecule. The shampoos may further include conventional ingredients, for example opacifying agents, sequestering agents, thickening agents, foam builders, builders and antibacterials.

US 3,988,438 discloses a hair shampoo with cleansing as well as conditioning properties, the composition of which comprises a polysaccharide, namely an alginate salt (preferably the triethylamine (TEA) salt of alginic acid), in addition to various cleansing agents and additives such as solvents, stabilisers, preservatives, sequestrants, perfume and colorants.

EP 0 591 443 discloses the use of oligosaccharides, namely gluco-oligosaccharides, fructo-oligosaccharides, galacto-oligosaccharides and mixtures thereof, that are easily metabolised by beneficial strains of skin or vaginal microflora, for the manufacture of a composition for the treatment of the skin or of the vaginal mucous membrane, aiming to favour the development of said beneficial microflora vis à vis pathogenic or non-desirable microflora strains. The composition, which may comprise from 0.1 to 20% and even more of the oligosaccharide, is typically in the form of a shampoo, liquid soap or body milk.

US 4,364,837 discloses a freely pourable shampoo composition comprising 20 to 75% water, 0.1 to 30% of at least one non-ionic or cationic conditioning agent, 3 to 60% of an anionic detergent and 15 to 70% of a water-miscible saccharide. Said saccharide includes mono- and disaccharides, polysaccharide molecules with chains comprising mainly glucose and containing at least three or more units of glucose, as well as hydrogenation products and mixtures thereof. High fructose syrup, made by enzymatic conversion of glucose to fructose, is a suitable saccharide too. The saccharides should have dextrose equivalents of about 20 to 100. A drawback of the shampoo composition is its high content (15 to 70%) of saccharide.

US 6,261,578 B1 discloses cosmetic rinse-off compositions for the treatment of the skin and the hair, that comprise a combination of a polysaccharide, including inulin but preferably guar gum, and an acrylic terpolymer composed of a monomer consisting of a carboxylic acid that contains an α,β-monoethylenic unsaturation, a non-ionic monomer bearing a urethane group, and a non-surfactant monomer with monoethylenic unsaturation which is different from the first mentioned monomer. The combination of the polysaccharide and said terpolymer provides desired thickening to the cosmetic composition in combination with desirable cosmetic performances. However, according to WO 02/055034 A2 (p.1, lines 17-19) and WO 02/055036 A2 (p.1, lines 18-21), said compositions still present disadvantages including insufficient rinse-off, insufficient stability at acidic pH, insufficient repartition on keratinous materials, and/or insufficient cosmetic properties.

WO 02/055034 A2 discloses cosmetic rinse-off compositions for the treatment of the hair and the skin that contain in a cosmetically acceptable medium, a combination of at least one fructan, preferably inulin, at least one other polysaccharide and at least one agent that is beneficial for the treatment of keratin materials. The compositions have a velvety-smooth texture, are easily rinsed off, and provide to the hair and the skin a soft feeling. However, they have the disadvantage requiring at least two different polysaccharides to provide the desired properties. This feature complicates the composition and manufacture of the rinse-off compositions, which obviously results in technical and economical drawbacks.

WO 02/055036 A2 discloses cosmetic rinse-off compositions that comprise in a cosmetically acceptable medium, a combination of a cationic polymer and at least a fructan. The compositions improve the detangling of the hair, provide soft feeling to treated keratinous material such as skin and hair, and are easily rinsed off.

In spite of the fact that cosmetic rinse-off compositions are extensively used, most of the prior art compositions nevertheless present one or more disadvantages or drawbacks. Typical disadvantages include a rather poor biodegradability of widely used ingredients, particularly of cationic polymers and acrylate-containing polymeric thickeners.

Besides, most rinse-off compositions present further disadvantages resulting from the presence of cationic polymers. In spite of their highly desirable properties as conditioning agent, cationic polymers namely present undesirable eutrophic effects on the surface waters which results from their nitrogen content and the huge amounts of cationic polymers that are yearly used in cosmetic rinse-off compositions. Accordingly, cationic polymers become less desirable as conditioning agents and there is even a tendency to restrict the use of cationic polymers in cosmetic rinse-off compositions.

Accordingly, industry is continuously searching for alternative and/or improved cosmetic rinse-off compositions that preferably are composed of easily biodegradable ingredients which do not contribute to eutrophication of the surface waters.

### Aim of the invention

The present invention aims to provide alternative cosmetic rinse-off compositions for the treatment of the hair and/or the skin, that preferably are free from one or more disadvantages or drawbacks of conventional rinse-off compositions.

### Description of the invention

Searching for alternative rinse-off compositions, the inventors found that fructan-type saccharides, in particular inulin-type fructans, are suitable as conditioning agents, and suitable as a component for the manufacture of aqueous cosmetic rinse-off compositions presenting desirable conditioning effects and desirable properties. Furthermore, the inventors surprisingly found that the use of inulin-type fructans enables the total substitution of cationic polymeric conditioning agents (cationic polymers) in conventional aqueous cosmetic rinse-off compositions.

For the sake of convenience, herein the constituents of the rinse-off compositions in accordance with the present invention are named components. In contrast herewith, as mentioned above, the constituents of conventional rinse-off compositions are named herein ingredients. Furthermore, with respect to compositions, % (percent) hereinafter refers to weight percent (wt%).

Accordingly, in a first aspect, the present invention relates to the use of an inulin-type fructan for the manufacture of a cosmetic rinse-off composition, characterised in that the rinse-off composition comprises from 0.10 to 10%, preferably 0.5 to 5%, most preferably 1 to 3%, of the inulin-type fructan (% in weight percent dry matter, calculated on the total weight of the rinse-off composition), and is free from (i) cationic polymeric conditioning agents, (ii) terpolymers composed of a monomer consisting of a carboxylic acid containing an a,β-monoethylenic unsaturation, a non-ionic monomer bearing a urethane group, and a non-surfactant monomer with monoethylenic unsaturation which is different from the first mentioned monomer,(hereinafter referred to in short "terpolymers"), and (iii) polysaccharides that are different from inulin-type fructan.

In a typical embodiment, the rinse-off composition is also free from monosaccharides and from di- and oligosaccharides that are different from inulin-type fructan, apart from the mono- and disaccharides that naturally occur in inulin-type fructan. Accordingly, fructose, glucose, sucrose and difructose dianhydride may be present in the rinse-off compositions in accordance with the present invention.

Conventionally oligosaccharide refers to saccharide molecules with degree of polymerisation (DP) up to 10, whereas polysaccharide refers to saccharide molecules with a DP above 10.

Apart from the inulin-type fructan, said components of the rinse-off compositions in accordance with the present invention are constituted by one or more ingredients in conventional amounts of conventional rinse off compositions, with the exception of the above restrictions.

The inulin-type fructan can be completely dissolved or only partly dissolved in the rinse-off composition, without that this substantially affects the kind and efficacy of the conditioning effects of the rinse-off composition.

In a preferred embodiment, the inulin-type fructan is typically used as a conditioning agent.

In a further preferred embodiment, the inulin-type fructan is used as a total substitute for cationic polymeric conditioning agents (cationic polymers) in conventional cosmetic rinse-off compositions. Although the weight ratio cationic polymers:inulin-type fructan for this substitution is not critical, the ratio is preferably within the range from 1:1 to 1:10.

In a second aspect, the present invention relates to a process for the manufacture of a cosmetic rinse-off composition, characterised in that 0.10 to 10%, preferably 0.5 to 5%, most preferably 1 to 3%, of an inulin-type fructan (% in weight percent dry matter, calculated on the total weight of the rinse-off composition), water and one or more other components constituted by one or more ingredients in conventional amounts of conventional cosmetic rinse-off compositions, with the exception of (i) cationic polymeric conditioning agents, (ii) terpolymers as defined hereinabove, and (iii) polysaccharides different from inulin-type fructans, are co-mixed.

In a typical embodiment, neither the inulin-type fructan component nor said other components include monosaccharides or di- and oligosaccharides that are different from inulin-type fructan, apart from the mono- and disaccharides that naturally occur in inulin-type fructan.

The process according to the present invention can be carried out according to conventional techniques. Co-mixing of the components can be carried out either batch-wise or in a continuous process, such as through in-line mixing.

Batch-wise co-mixing can, for example, be carried out as follows: to a preset amount of water, the components can be added separately or simultaneously under stirring, and stirring is continued till a homogeneous composition is obtained. To avoid excessive foam formation, the mixture is stirred slowly. Stirring is typically carried out by means of an anchor agitator rotating at low speed in a reactor with only small mixing baffles. Although the order of addition of the components is not critical, it is recommended, in order to avoid excessive foam formation, to follow the order commonly used for the manufacture of conventional rinse-off compositions with the concerned ingredients. Accordingly, usually a preset amount of water is charged with the components, apart from the foam-forming surfactants and the thickener. After homogenising of said mixture, subsequently the surfactant and finally the thicker are added under stirring at low speed till the mixture is homogeneous.

The co-mixing according to the process of the present invention may optionally be carried out by co-mixing one or more components or part of said components with one or more premix compositions that contain the remaining components and/or the complementary part of the former components, or by co-mixing two or more pre-mix compositions. In the co-mixing process, water is considered as a component, which optionally may partly or wholly be included in one or more of the premixes. The use of a pre-mix may be preferred and be beneficial for practical and/or technical reasons.

It may sometimes be beneficial to warm up the mixture or the pre-mix in order to increase the solubility of one or more of the components, and/or to facilitate the co-mixing. The maximal temperature to which a mixture or a premix may be warmed up is known to the skilled person or can easily be determined by routine experiments.

Also the appropriate apparatus, the manner and duration of stirring to obtain the desired homogeneous mixture are well known in the art or can be determined by the skilled person without undue burden by routine experiments.

In a third aspect, the present invention relates to cosmetic rinse-off compositions, characterised in that they contain 0.10 to 10%, preferably 0.5 to 5%, most preferably 1 to 3%, of an inulin-type fructan (% in weight percent dry matter, calculated on the total weight of the rinse-off composition), water and one or more other components constituted by one or more ingredients in conventional amounts of conventional cosmetic rinse-off compositions, with the exception of (i) cationic polymeric conditioning agents, (ii) terpolymers as defined above, and (iii) polysaccharides that are different from inulin-type fructans.

In a typical embodiment, the cosmetic rinse-off composition is also free from monosaccharides and from di-and oligosaccharides that are different from inulin-type fructan, apart from the mono- and disaccharides that naturally occur in inulin-type fructan.

The rinse-off compositions in accordance with the present invention can appear, dependent on their composition, as a solution, a mixture of two liquid phases, an emulsion, or a suspension. They present an acceptable physico-chemical stability and typically appear in the form of a homogeneous, pourable or free flowing liquid, such as a hair-shampoo, hair-conditioner, shower-shampoo, shower-gel or bath-foam.

Fructan is composed of saccharides consisting of polyfructose molecules that may or may not bear one terminal glucose unit. Fructan are subdivided into levan and inulin.

Levan-type fructan is composed of polyfructose molecules wherein the fructose units are mostly connected to each other by β(2-6) fructosyl-fructose linkages. Levan is synthesised by certain plant species and by certain bacteria.

Inulin-type fructan is composed of polyfructose molecules of which the fructose units are exclusively or mainly connected to each other by ß(2-1) fructosyl-fructose linkages. The polyfructose molecules can be linear, namely when all the fructose units are exclusively connected to each other by ß(2-1) fructosyl-fructose linkages, but can also be branched, namely when the fructose units are connected to a certain extent but for less than 50 percent to each other by ß(2-6) fructosyl-fructose linkages. The polyfructose molecules are represented by the general formulae GFn and Fm, wherein G represents a glucosyl unit, F a fructosyl unit, and n and m are integers that indicate the number of fructosyl units in the molecule. The values n+1 and m are referred to as degree of polymerisation, represented by DP, and are a characteristic of the polyfructose molecules of the inulin-type fructan.

Inulin-type fructan is well known in the art. It is synthesised by many plant species, can originate from bacterial activity, can be enzymatically synthesised in vitro, for example from sucrose, and can be obtained by partial hydrolysis of large inulin molecules of bacterial and plant origin. Inulin-type fructan naturally occurs as a polydisperse mixture of linear and/or branched polyfructose molecules that is characterised *inter alia* by the number-average degree (in short average degree) of polymerisation, indicated as av. DP or DP, of the polyfructose molecules. The DP and the av. DP of inulin-type fructan are dependent on the origin of the fructan.

Inulin-type fructan composed of molecules with a DP ranging from 2 to 10 is conventionally and interchangeably named oligofructose, fructo-oligosaccharide or inulo-oligosaccharide. Inulin-type fructan composed of molecules with a DP ranging from 2 to about 100,000 is commonly named inulin. Accordingly, by inulin-type fructan are meant herein both inulin and oligofructose.

Inulin from plant origin has a DP ranging from 2 to about 200, mostly from 2 to about 100. Inulin is mostly isolated from roots of chicory (Cichorium intybus), from tubers of Jerusalem artichoke (Helianthus tuberosus) and Dahlia, and from the head (piña) of the Blue Agave. In the native form, namely without a treatment to increase or reduce the DP, chicory inulin has a DP ranging from 2 to about 70 and an av. DP of about 10 to about 12; J. artichoke inulin a DP ranging from 2 to about 40 and an av. DP of about 6; dahlia inulin an av. DP of about 15 to about 20; and agave inulin an av. DP of about 14 to about 18.

Chicory inulin is slightly branched (containing about 2 to 5% branching), whereas agave inulin is highly branched.

Inulin from bacterial origin is usually highly branched and has a high DP, commonly ranging from about 10,000 to about 100,000.

Enzymatically synthesised inulin-type fructan mostly has a DP lower than 10, typically a DP ranging from 3 to 5.

Oligofructose obtained by partial hydrolysis of inulin molecules typically has a DP ranging from 2 to about 9.

At commercial scale, inulin is mainly manufactured from chicory roots. Chicory inulin is for example available as a spray-dried powder in various grades from ORAFTI S.A. (Belgium), under the trade name RAFTILINE®. Typical RAFTILINE® grades include ST (= standard grade with a DP ranging from 2 to about 70 and an av. DP 10 to 12, and containing in total about 8% glucose, fructose and sucrose), GR (= standard grade in the form of a granulated powder), LS (= low sugar grade with av. DP 10 to 12, but containing in total less than 1% glucose, fructose and sucrose), HP (= high performance grade, namely long-chain inulin with a DP ranging from 10 to about 70 and an av. DP of at least about 23, which is essentially free of glucose, fructose and sucrose).

Agave inulin with an av. DP of about 14 to 16 is for example available under the trade name GAVEDIET® PR from Industrias Colibri Azul S.A. de C.V.,(Mexico).

Oligofructose can be obtained at industrial scale by partial (acidic or enzymatic) hydrolysis of inulin from plant origin or from bacterial origin, or by enzymatic synthesis in vitro according to well-known techniques.

Oligofructose obtained by partial, enzymatic hydrolysis of chicory inulin is for example available in several grades from ORAFTI S.A. (Belgium), under the trade name RAFTILOSE®. Typical grades include RAFTILOSE® P95 (spray-dried powder with about 97% dry matter (d.m.) containing ≥ 93% (on d.m.) oligofructose with a DP from 2 to 9, mainly from 2 to 7, and maximally about 7% in total of glucose, fructose and sucrose), RAFTILOSE® L95 (aqueous liquid containing about 75% dry matter with a d.m. composition corresponding to the one of RAFTILOSE® P95), and RAFTILOSE® L85 (aqueous liquid containing about 75% dry matter containing about 85% (on d.m.) oligofructose of DP ranging from 2 to about 9, mainly from 2 to about 7, and in total about 5 to 10% glucose and fructose, and about 5 to 9% sucrose.

Oligofructose with DP from 3 to about 5 obtained by in vitro enzymatic synthesis is for example available under the trade names ACTILIGHT® and NEOSUGAR® from Beghin-Meiji, (France/Japan), and Nutraflora® from GTC, USA.

All said inulin-type fructans as well as their commercial grades are suitable for use in accordance with the present invention.

In a preferred embodiment of the invention, the inulin-type fructan is oligofructose, preferably oligofructose with a DP ranging from 2 to 9, typically from 2 to 7 or 3 to 5. Highly preferred oligofructose is obtained by enzymatic hydrolysis from chicory inulin, for example the commercial grade products available under the trade name RAFTILOSE® from ORAFTI S.A. (Belgium).

In another preferred embodiment of the invention, the inulin-type fructan is inulin with a DP ranging from 2 to about 200, more preferably inulin with a DP ranging from 2 to about 100, typically inulin from plant origin with a DP ranging from 2 to about 100.

In a highly preferred embodiment of the invention, the inulin-type fructan is chicory inulin. Preferred grades of inulin in accordance with the present invention include chicory inulin with a DP range from 2 to about 70, for example the commercial products RAFTILINE® ST, GR and LS, as well as long-chain chicory inulin with a DP range from about 10 to about 70 and an av. DP of 20 to 25, for example the commercial product RAFTILINE® HP (RAFTILINE®: trade name of ORAFTI S.A., Belgium).

In a further preferred embodiment, the inulin-type fructan is inulin from Jerusalem artichoke or inulin from Dahlia.

In still another preferred embodiment of the invention, the inulin-type fructan is a branched inulin, for example agave inulin, typically inulin from blue agave with an av. DP of about 14 to 18, such as the commercial product GAVEDIET® PR (trade name of Industrias Colibri Azul S.A., Mexico).

Said other components constituted by the ingredients of conventional rinse-off compositions and the amounts thereof that are commonly used are well-known to the skilled person. Alternatively, suitable ingredients and the suitable amounts thereof for particular rinse-off compositions in accordance with the present invention can be routinely selected and determined from the conventional ingredients by the skilled person without undue burden. Typically these conventional ingredients comprise, for example, apart from water, one or more compounds from one or more of the classes including detergents, cleaning agents, and foam builders, such as soaps, anionic surfactants, non-ionic surfactants, amphoteric surfactants, and mixtures thereof; foam stabilisers; preservatives; anti-bacterial agents; stabilising agents; agents that inhibit colour deterioration of the rinse-off compositions; sequestering agents; builders; thickening agents; colouring agents; opacifying agents; and perfuming agents. These conventional ingredients are well known in the art, and for example disclosed in WO 02/055034, WO 02/055036 and US 4,364,837.

The present invention is further illustrated by the examples given below.

### Example 1.

### Preparation of hair shampoo rinse-off compositions.

General procedure: Rinse-off compositions according to the present invention with different grades of inulin-type fructan were prepared by conventional techniques and equipment, as follows:
- water, an inulin-type fructan, PEG-7 glyceryl cocoate and PEG-200 hydrogenated glyceryl palmate were mixed and homogenised, forming premix 1 (phase A),
- separately from premix 1, all other components were mixed and homogenised, forming premix 2 (phase B),
- then, premix 1 and premix 2 were combined and homogenised, and the pH was adjusted to the desired value (phase C), forming a pourable hair shampoo.
According to said general procedure also comparative hair shampoo compositions have been prepared wherein the inulin-type fructan was substituted either by saccharides that are different from the inulin-type fructan, or by a cationic polymer, namely Polyquaternium-10 (UCARE® Polymer JR-400, Trade name of Amerchol, USA).

The compositions of the prepared hair shampoo's are indicated in Table 1 below.

**Table 1**

| | |
|---|---|
| 30% | ammonium lauryl sulfate (primary tenside) (Ufarol AM30: trade name of UNGER, Norway) |
| 7% | cocoamidopropul hydroxysultaïne (secunday tenside) (Qovaq HSLS: trade mark of GOVA, Belgium) |
| 4% | Saboderm® SHO (= 35% PEG-7 glyceryl cocoate, 35% PEG-200 hydrogenated glyceryl palmate, 30% water)(thickener and foam forming agent) (Saboderm®: trade name of SABO, Italy) |
| 1% | Phenochem®: (phenoxyethanol + methyl-, ethyl-, propyl-, butylparaben) (preservative), (Phenochem®, trade name of SHARON, Israel) |

+ conditioning agent(% as indicated), being either:
- inulin-type fructan, or
- cationic polymer, Polyquaternium-10(Ucare® Polymer JR-400; trade name of Amerchol, USA), or
- saccharide different from the inulin-type fructan,
+ citric acid, amount to reach a desired pH of 5.5
+ water, quantity to complete the composition to 100%.

### Example 2

### Comparison of hair shampoo rinse-off compositions.

The foam formation of compositions prepared in Example 1 was tested by the following general method: into 1 litre shampoo in a 5 litre beaker, nitrogen was blown at a preset rate during 30 seconds, and the foam formed was measured (height in cm of the foam). The examined shampoo's and results are indicated in Table 2 below.

**Table 2**

| Conditioning agent | Raftiline® | | | Raftiline® | | Raftilose® | |
|---|---|---|---|---|---|---|---|
| | GR | | | HP | | P95 | |
| Conc. | 0% * | 5% | 10% | 0%* | 10% | 0%* | 10% |
| Foam (cm) | 14 | 12 | 9 | 14 | 14 | 14 | 14 |

| | | | | | | | |
|---|---|---|---|---|---|---|---|
| *: comparative example: inulin-type fructan replaced by 0.5% Polyquaternium-10 | | | | | | | |

From the results it follows that shampoo compositions wherein the Polyquaternium-10 conditioning agent has been completely substituted by an inulin-type fructan still maintain good foam forming properties, certainly at a concentration of 5%, and for certain fructans even up to a concentration of 10%.

### Example 3

The functionality of several of the rinse-off compositions according to Example 1 was examined by panel testing. A rating was given based on visual and sensorial evaluation of the treated hair. The results are given in Table 3.

**Table 3**

| Conditioning Component | % | Dry Feeling | Volume | Gloss |
|---|---|---|---|---|
| *Polyquat-10 | 0.5 | 0 | 0 | 0.5 |
| ST | 1 | 0.5 | 0.5 | 0 |
| ST | 2 | 1 | 1 | 0 |
| ST | 5 | 1 | 1 | 0 |
| ST | 10 | 1 | 1 | 0 |
| P95 | 1 | 0.5 | 0.5 | 0 |
| P95 | 2 | 1 | 3 | 0 |
| P95 | 5 | 1 | 2 | 0 |
| P95 | 10 | 1 | 1 | 0 |
| *Raftisweet® | 2 | 0.1 | 0 | 0 |
| *Sucrose | 2 | 0 | 0 | 0 |
| *Maltodextrin(1) | 2 | 0 | 0 | 0 |
| *Maltodextrin(2) | 2 | 0 | 0 | 0 |

| | | | | |
|---|---|---|---|---|
| *: comparative | | | | |

ST: Raftiline® ST (trade name, Orafti S.A., Belgium)
P95: Raftilose® P95 (trade name, Orafti S.A., Belgium)
Raftisweet®: Raftisweet® F85/75 (a 85% fructose/15% glucose syrup at 75% dry weight)(trade name, Orafti S.A., Belgium)
Polyquat-10: Ucare®Polymer JR-400; trade name, Amerchol USA
Maltodextrin(1): Maltrin QD M440:trade name of Grain Processing Corp.[GPC, USA] for maltodextrin of DE 5
Maltodextrin(2): Glucidex 19D: Trade name of Roquettes Frères (France) for maltodextrin of DE 19
Legend of scores: 0:normal, neutral; +1:good; +2: better; +3:very good; -1:bad; (and in between scores).

It was found that glucose, fructose, sucrose and maltodextrins have no effects whereas inulin-type fructan provides in general desirable conditioning effects, particularly good dry feeling and volume.

### Example 4

### Preparation of hair shampoo rinse-off compositions.

According to the general procedure of example 1 shampoo compositions were prepared containing inulin-type fructan or cationic polymer* Polyquaternium-10 (*: comparative).

The compositions of the shampoo's is as follows:
- 30%: ammonium lauryl sulfate (primary tenside)
(Ufarol AM30: trade name of UNGER,Norway)
- 7%: cocamidopropyl hydroxysultaïne (secundary tenside)
(Qovaq HSLS: trade mark of GOVA, Belgium)
- 4%: Saboderm SHO: trade name of SABO, Italy (= 35% PEG-7 glyceryl cocoate, 35% PEG-200 hydrogenated glyceryl palmate, 30% water)(thickener and foam forming agent)
- 1%: Phenochem®: (phenoxyethanol + methyl-, ethyl-, propyl-, butylparaben), (Phenochem®, trade name of SHARON, Israel)
- 0.3%: yogurt powder (Yogurtene®; trade name of QUEST, UK)
- 0.2%: PEG-14 M (Polyox®; trade name of Union Carbide)
- 0.3%: bishydroxyethyl biscetylmalonamide (Questamid® H, trade name of QUEST, UK)
- 0.5%: apricot kernal oil (Henry Lamotte, Germany)
- 0.15%: Naturein casein peptide (QUEST,UK)
- 0.2%: Naturein rice peptide (QUEST,UK)
- 0.5%: Vegebios of grape fruit (SOLABIA, France)
- 0.5%: glycolysate of white nettle (SOLABIA, France)
- 0.5%: lemon glycolysate (SOLABIA, France)
+ (as indicated in Table 4 below) inulin-type fructan Raftiline® GR (ORAFTI S.A., Belgium) or Polyquaternium-10 (Ucare® Polymer JR-400; trade name of Amerchol, USA)
+ water (quantity to complete the composition to 100%).

The functionality of the rinse-off compositions was examined and rated as indicated in Example 3. The results are given in Table 4 below.

**Table 4**

| Conditioning | % | combability | Feeling | | Volume | Gloss |
|---|---|---|---|---|---|---|
| component | | (wet) | Wet | Dry | | |
| Polyquaternium-10 * | 1 | 0.5 | 0 | 0 | 0 | 0 |
| RAFTILINE® GR | 1 | 1 | 0 | 1 | 0.5 | 0 |
| RAFTILINE® GR | 2 | 0.5 | 0 | 1 | 1 | 0 |
| RAFTILINE® GR | 5 | 0 | 0 | 1 | 1 | 0 |

| | | | | | | |
|---|---|---|---|---|---|---|
| *: comparative Legend of scores: 0:normal, neutral; +1:good; (and in between scores). | | | | | | |

The results show that by complete substitution of a cationic polymeric conditioning agent by an inulin-type fructan, particularly chicory inulin of DP ranging from 2 to about 70 and av. DP of about 10-12, rinse-off compositions can be formulated that are suitable as shampoo, presenting inter alia good foaming properties and good conditioning properties.

Very suitable concentrations of inulin-type fructan are from 1 to 5%, optimally about 2%.

### Example 5

### Preparation and evaluation of hair shampoo's

A hair shampoo according to the present invention and a corresponding conventional composition, referred to, respectively, as Shampoo A and Shampoo B, were prepared by the general procedure outlined in Example 1 with compositions given in Table 5 below. Lactic acid instead of citric acid was used to reach the desired pH.

**Table 5**

| Shampoo composition | | | |
|---|---|---|---|
| Manufact-phase | Components | Formula A | Formula B* |
| | | (%) | (%) |
| A | water | ad 100 | ad 100 |
| | ammonium | 35 | 35 |
| | laurylsulfate | | |
| | cocamidopropyl | 8 | 8 |
| | hydroxysultaine | | |
| | Raftilose® L95(1) | 2 | 0 |
| | polyquaternium-10(2) | 0 | 0.3 |
| | PEG-14M (3) | 0.09 | 0.09 |
| B | PEG-200 hydrogenated | 3.5 | 3.5 |
| | glyceryl palmate ; | | |
| | glyceryl cocoate | | |
| | Perfume True | 0.4 | 0.4 |
| | Pantene(4) | | |
| C | Lactic acid | to pH 4.5-5 | to pH 4.5-5 |

| | | | |
|---|---|---|---|
| *: comparative | | | |
| (1): trade name ORAFTI S.A., Belgium | | | |
| (2): Ucare® Polymer JR-400; trade name of Amerchol, USA | | | |
| (3): Polyox®; trade name of Union Carbide, USA | | | |
| (4): trade name of Luzi, Switzerland | | | |

The functionality of the shampoo's according to the invention was evaluated in a "Half-head test" in comparison with a corresponding shampoo composition containing the cationic polymer Polyquaternium-10 instead of the inulin-type fructan. The evaluation was carried out as a double blind study with a group of 10 subjects who were chosen to include a range of hair lengths and styles, as indicated in Table 6 below.

**Table 6**

| Hair types of the panel members | | | |
|---|---|---|---|
| Head number | Hair type | | |
| 1 | short | normal | untreated |
| 2 | short | normal | permanent wave |
| 3 | short | dry | semi-permanent colour |
| 4 | short | dry | highlights |
| 5 | short | normal | permanent wave |
| 6 | medium | greasy | untreated |
| 7 | short | dry | untreated |
| 8 | short | dry | untreated |
| 9 | long | normal | untreated |
| 10 | medium | normal | semi-permanent colour |

The tests were "half head tests", namely tests wherein the half of the subjects received shampoo A on the left side of the head and shampoo B on the right side, and the second half of the subjects received the shampoo's applied to the opposite sites.

The scoring system is based on a scale of 0-100. Higher scores indicate better performances, with the exception of the wet hair feeling. For the latter the optimum score is 50, with a greasy over-conditioned feel attributed a score 0, and a harsh over-washed feel a score 100.

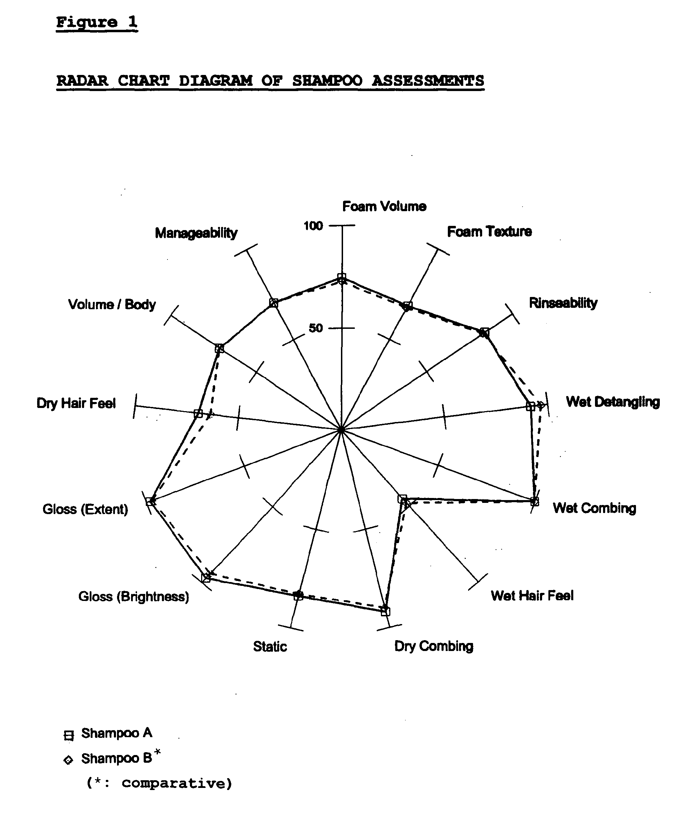

The scores for each of the assessed parameters are given below in Table 7, together with a calculated mean value. These mean values have also been plotted onto a radar chart diagram, shown in Figure 1 below, enabling easy comparisons.

**Table 7**

| Shampoo assessments | | | | | | | | | | | |
|---|---|---|---|---|---|---|---|---|---|---|---|
| Shampoo A | | | | | | | | | | | |
| Head No | 1 | 2 | 3 | 4 | 5 | 6 | 7 | 8 | 9 | 10 | Mean |
| Foam Volume | 80 | 80 | 60 | 80 | 80 | 80 | 80 | 80 | 80 | 90 | 75 |
| Foam Texture | 70 | 80 | 70 | 80 | 60 | 70 | 60 | 80 | 60 | 60 | 69 |
| Rinseability | 100 | 80 | 80 | 80 | 80 | 100 | 80 | 80 | 80 | 80 | 84 |
| Wet Detangling | 100 | 80 | 100 | 100 | 100 | 80 | 100 | 100 | 80 | 80 | 92 |
| Wet Combing | 100 | 100 | 100 | 100 | 100 | 100 | 100 | 100 | 100 | 100 | 100 |
| Wet Hair Feel | 40 | 70 | 50 | 70 | 50 | 30 | 30 | 30 | 30 | 50 | 45 |
| Dry Combing | 100 | 100 | 100 | 70 | 90 | 100 | 100 | 80 | 80 | 100 | 92 |
| Static | 100 | 100 | 100 | 100 | 50 | 80 | 100 | 100 | 70 | 40 | 84 |
| Gloss (Brightness) | 100 | 100 | 80 | 90 | 100 | 100 | 100 | 100 | 100 | 100 | 97 |
| Gloss (Extent) | 100 | 100 | 100 | 80 | 100 | 100 | 100 | 100 | 100 | 100 | 98 |
| Dry Hair Feel | 90 | 60 | 70 | 70 | 60 | 90 | 80 | 80 | 70 | 60 | 69 |
| Volume / Body | 80 | 70 | 80 | 70 | 80 | 70 | 80 | 80 | 80 | 60 | 71 |
| Manageability | 80 | 70 | 80 | 70 | 60 | 70 | 80 | 80 | 60 | 60 | 71 |
| | | | | | | | | | | | |

| Shampoo B* | | | | | | | | | | | |
|---|---|---|---|---|---|---|---|---|---|---|---|
| Head No | 1 | 2 | 3 | 4 | 5 | 6 | 7 | 8 | 9 | 10 | Mean |
| Foam Volume | 70 | 80 | 50 | 80 | 70 | 60 | 80 | 80 | 70 | 90 | 73 |
| Foam Texture | 60 | 70 | 60 | 70 | 70 | 60 | 70 | 80 | 70 | 70 | 68 |
| Rinseability | 100 | 80 | 80 | 80 | 80 | 90 | 80 | 80 | 80 | 80 | 83 |
| Wet Detangling | 100 | 100 | 100 | 100 | 100 | 100 | 100 | 100 | 70 | 100 | 97 |
| Wet Combing | 100 | 100 | 100 | 100 | 100 | 100 | 100 | 100 | 100 | 100 | 100 |
| Wet Hair Feel | 40 | 80 | 50 | 70 | 50 | 30 | 30 | 40 | 40 | 50 | 48 |
| Dry Combing | 100 | 80 | 100 | 70 | 90 | 100 | 100 | 80 | 80 | 100 | 90 |
| Static | 100 | 80 | 100 | 80 | 50 | 80 | 100 | 100 | 80 | 60 | 83 |
| Gloss (Brightness) | 100 | 100 | 80 | 80 | 80 | 100 | 100 | 100 | 100 | 100 | 94 |
| Gloss (Extent) | 100 | 100 | 100 | 70 | 100 | 100 | 100 | 100 | 100 | 100 | 97 |
| Dry Hair Feel | 90 | 40 | 60 | 50 | 70 | 70 | 50 | 70 | 60 | 70 | 63 |
| Volume / Body | 80 | 80 | 80 | 70 | 60 | 60 | 80 | 80 | 50 | 70 | 71 |
| Manageability | 80 | 80 | 80 | 70 | 60 | 60 | 80 | 80 | 50 | 70 | 71 |

| | | | | | | | | | | | |
|---|---|---|---|---|---|---|---|---|---|---|---|
| (*: comparative) | | | | | | | | | | | |

Figure 1: Radar chart diagram of "half-head test" evaluation on a group of 10 subjects of hair shampoo containing inulin or Polyquaternium-10.
From Figure 1 it follows that inulin-type fructan is suitable for the manufacture of effective rinse-off compositions and is suitable to completely substitute cationic polymeric conditioning agents in rinse-off compositions. Furthermore, Figure 1 shows that there is very little difference in functionality between hair rinse-off compositions containing inulin-type fructan or cationic polymer Polyquaternium-10.

From the above examples it clearly follows that inulin-type fructan can be used for the manufacture of cosmetic rinse-off compositions for the treatment of the hair and/or the skin in accordance with the present invention, and that said compositions are effective cosmetic rinse-off compositions presenting a functionality that is comparable with the one of a conventional standard composition containing the cationic polymer Polyquaternium-10.

Furthermore, inulin-type fructan appears to be suitable as a total substitute for cationic polymeric conditioning agents in cosmetic rinse-off compositions. A higher amount (wt%) of inulin-type fructan than that of the cationic polymers may have to be used to obtain a comparable functionality. However, said higher amount of inulin-type fructan compared to cationic polymers is compensated by several advantages resulting from the use of the inulin-type fructan instead of cationic compounds.

Indeed, the rinse-off compositions in accordance with the present invention are free from the less desirable cationic polymers, many of which are poorly biodegradable, have undesirable eutrophic effects, and/or are to a certain extent cytotoxic. Unlike cationic polymers, the inulin-type fructan are non-toxic, are easily biodegradable, and neither the fructan nor its degradation products present eutrophic effects.

A further advantage of the use of inulin-type fructan resides in the availability of various grades, which enables to select without undue burden by routine experiments the most appropriate inulin-type fructan for the intended use or composition, for example by taking into account the DP range, the av. DP and/or the solubility in aqueous medium or aqueous rinse-off medium.

Furthermore, the availability of various inulin-type fructan grades with different DP ranges enables to slightly adjust the viscosity of the rinse-off composition by selecting the grade of the inulin-type fructan that is used in the rinse-off composition.

Still a further advantage resides in the fact that various commercial grades of inulin-type fructan are readily available at affordable cost and in that they are a renewable source material.

## Claims

1. Use of an inulin-type fructan for the manufacture of a cosmetic rinse-off composition for the treatment of the hair and/or the skin, **characterised in that** the rinse-off composition, apart from conventional ingredients of rinse-off compositions in conventional amounts, comprises from 0.10 to 10% of the inulin-type fructan (% in weight percent dry matter, calculated on the total weight of the rinse-off composition), and is free from (i) cationic polymeric conditioning agents, (ii) terpolymers composed of a monomer consisting of a carboxylic acid containing an α,β-mono-ethylenic unsaturation, a non-ionic monomer bearing a urethane group, and a non-surfactant monomer with monoethylenic unsaturation which is different from the first mentioned monomer,(hereinafter "terpolymers"), and (iii) polysaccharides that are different from inulin-type fructan.

2. Use according to claim 1, **characterised in that** the rinse-off composition is also free from monosaccharides and from di- and oligosaccharides that are different from inulin-type fructan, apart from the mono- and disaccharides that naturally occur in inulin-type fructan.

3. Use according to claim 1 or claim 2, **characterised in that** the inulin-type fructan is used as conditioning agent.

4. Use according to any one of claims 1 to 3, **characterised in that** the inulin-type fructan is used as a complete substitute for cationic polymeric conditioning agents in conventional rinse-off compositions.

5. Use according to anyone of claims 1 to 4, **characterised in that** the rinse-off composition contains the inulin-type fructan at a concentration from 0.5 to 5%.

6. Use according to anyone of claims 1 to 5, **characterised in that** the inulin-type fructan is oligofructose.

7. Use according to claim 6, **characterised in that** the oligofructose has a DP ranging from 2 to 9.

8. Use according to claim 6, **characterised in that** the oligofructose as a DP ranging from 3 to 5.

9. Use according to anyone of claims 1 to 5, **characterised in that** the inulin-type fructan is inulin.

10. Use according to claim 9, **characterised in that** the inulin has a DP ranging from 2 to 100.

11. Use according to claim 9, **characterised in that** the inulin-type fructan is chicory inulin with a DP ranging from 2 to 70.

12. Use according to claim 9, **characterised in that** the inulin-type fructan is inulin from agave, Jerusalem artichoke or dahlia.

13. Process for the manufacture of a cosmetic rinse-off composition, **characterised in that** 0.10% to 10% weight (calculated on the total weight of the rinse-off composition) of an inulin-type fructan defined in anyone of claims 1 and 6 to 12, water and one or more other components constituted by one or more ingredients in conventional amounts of conventional rinse-off compositions, with the exception of (i) cationic polymeric conditioning agents, (ii) terpolymers as defined in claim 1, and (iii) polysaccharides that are different from inulin-type fructan, are co-mixed.

14. Process according to claim 13, **characterised in that** neither the inulin-type fructan component nor said other components include monosaccharides or di- and oligosaccharides that are different from inulin-type fructan, apart from the mono- and disaccharides that naturally occur in inulin-type fructan.

15. Process according to claim 13 or claim 14, **characterised in that** it involves co-mixing of one or more components or part of said components with one or more premix compositions that contain the remaining components and/or the complementary part of the former components, or co-mixing of two or more pre-mix compositions.

16. Cosmetic rinse-off composition, **characterised in that** it contains from 0.10% to 10% weight (calculated on the total weight of the rinse-off composition) of an inulin-type fructan defined in any one of claims 6 to 12, water and one or more other components constituted by one or more ingredients in conventional amounts of conventional cosmetic rinse-off compositions, with the exception of (i) cationic polymeric conditioning agents, (ii) terpolymers as defined in claim 1, and (iii) polysaccharides that are different from inulin-type fructan.

17. Cosmetic rinse-off composition according to claim 16, **characterised in that** the rinse-off composition is also free from monosaccharides and from di- and oligosaccharides that are different from inulin-type fructan, apart from the mono-and disaccharides that naturally occur in inulin-type fructan.

18. Cosmetic rinse-off composition according to claim 16 or claim 17, **characterised in that** it the inulin-type fructan is oligofructose.

19. Cosmetic rinse-off composition according to claim 16 or claim 17, **characterised in that** it the inulin-type fructan is inulin.

20. Cosmetic rinse-off composition according to claim 19, **characterised in that** the inulin has a DP ranging from 2 to 100.

21. Cosmetic rinse-off composition according to claim 20, **characterised in that** it the inulin-type fructan is selected from the group consisting of chicory inulin, agave inulin, dahlia inulin and Jerusalem artichoke inulin.
